# EUROPEAN PATENT APPLICATION

(11) **EP 2 397 135 A1**
(43) Date of publication of application: **21.12.2011**
(21) Application number: 10305653.7
(22) Date of filing: 18.06.2010
(51) Int. Cl.: A61K 31/282, A61K 31/337, A61K 31/555, A61K 33/24, A61K 45/06, A61K 31/167

(54) **An antitumoral combination comprising ombrabulin, a taxane derivative and a platinum derivative**

(71) Applicant: SANOFI, 75013 Paris (FR)
(72) Inventor: The designation of the inventor has not yet been filed
(74) Representative: Gaslonde, Aude

(57) **Abstract**

The invention concerns an antitumoral combination comprising ombrabulin, a taxane derivative and a platinum derivative and its use in the treatment of advanced solid tumors.

## Description

The invention concerns an antitumoral combination comprising ombrabulin, a taxane derivative and a platinum derivative and its use in the treatment of advanced solid tumors.

### [Prior art and problem to be solved]

WO 99/51246 discloses the ombrabulin/platinum salt combination.

WO 2004/037258 discloses the combination of ombrabulin with various antitumoral agents including taxanes (Taxol®, Taxotere®).

There is still a need to find and optimize new therapeutic options to treat patients with advanced solid tumors.

The invention meet this need by providing a new pharmaceutical antitumoral combination comprising ombrabulin, a taxane derivative and a platinum derivative for which doses of each component and a suitable administration protocol has been determined, to obtain a well tolerated combination which does not exacerbate the toxicity of each of the antitumoral agents and which allows the treatment of advanced solid tumors either by stabilizing or by inducing a partial or a complete regression of the tumor.

### [Description of the invention]

The invention concerns an antitumoral combination comprising ombrabulin, a taxane derivative and a platinum derivative, these therapeutic components being in the form of a free base or of an addition salt with a pharmaceutical acceptable acid, or in the form of a hydrate or of a solvate, where this antitumoral combination is well tolerated, does not exacerbate the toxicity of each of the antitumoral agents and which allows the treatment of advanced solid tumors either by stabilizing or by inducing a partial or a complete regression of the tumor.

Ombrabulin (AVE8062) belongs to the family of combretastatins and has the formula: 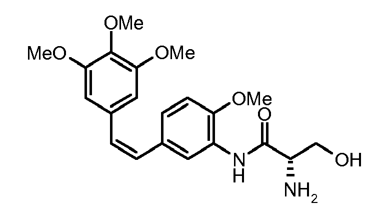 (it is the Z isomer)

It is an antivascular agent (or VDA, Vascular Disrupting Agent). It has the chemical name: (Z)-N-[2-methoxy-5-[2-(3,4,5-trimethoxyphenyl)vinyl]phenyl]-L-serinamide. This compound, which is described in EP 731085 B1, may be prepared according to the method described in WO 03/084919. Ombrabulin may be administered in base form (cf. above formula) or in the form of a salt of a pharmaceutically acceptable acid, for example in the form of the hydrochloride, represented below:

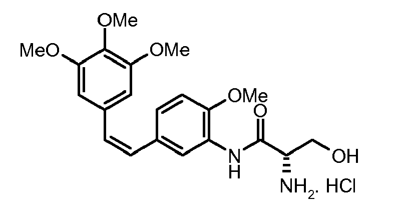

Once administered, ombrabulin releases *in vivo* the active metabolite (Z)-1-(3-amino-4-methoxyphenyl)-2-(3,4,5-trimethoxyphenyl)ethene, which has the formula:

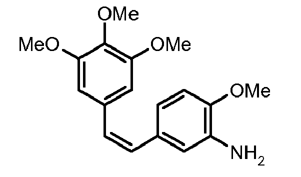

It is therefore also possible to substitute, for ombrabulin, another combretastatin of formula: 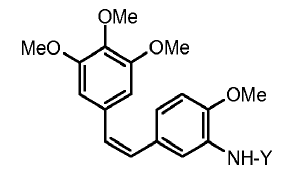 in base form or in the form of a salt of a pharmaceutically acceptable acid, in which Y represents an amino acid, which releases *in vivo* this metabolite.

The taxane derivative may for example be chosen from paclitaxel or docetaxel.

The platinum derivative may for example be chosen from cisplatin or carboplatin.

The combination comprises an effective quantity of ombrabulin, an effective quantity of a taxane derivative and an effective quantity of a platinum derivative.

Ombrabulin may be administered by perfusion at a dose comprised between 15 and 35 mg/m², for example chosen from the following doses: 15.5; 20 ; 25 ; 30 and 35 mg/m². Docetaxel may be administered by perfusion at a dose of 60 or 75 mg/m².
Paclitaxel may be administered by perfusion at a dose of 175 or 200 mg/m².
Cisplatin may be administered by perfusion at a dose of 75 mg/m².
Carboplatin may be administered by perfusion at a dose of AUC 5 and AUC 6.

Preferentially, ombrabulin may be used in combination with docetaxel and cisplatin or in combination with paclitaxel and carboplatin.

The cycle of administration of the three antitumoral agents is repeated with an interval between two administrations of three weeks.

The invention also concerns the use of ombrabulin, a taxane derivative and a platinum derivative for the preparation of an antitumoral combination here above disclosed.

The invention also concerns the above disclosed antitumoral pharmaceutical combination comprising ombrabulin, a taxane derivative and a platinum derivative, these agents being in the form of a free base or of an addition salt with a pharmaceutical acceptable acid, or in the form of a hydrate or of a solvate, for its use as a medicament in the treatment of advanced solid tumors.
Examples of solid tumors that may be treated with the combination of the invention are - but not exclusively - lung tumors, ovarian tumors and breast tumors including triple negative breast tumors.

### Definitions

- pharmaceutically acceptable acid: organic or inorganic acid having a low toxicity (see "Pharmaceutical salts" J.Pharm.Sci. 1977, 66, 1-19);
- effective amount: amount of a pharmaceutical compound that produces an effect on the treated tumour.
- advanced solid tumors: locally advanced or metastatic solid tumors ie tumors which are not operable any more.

The combination is administered repeatedly in a course of several cycles according to a protocol that depends on the nature and on the stage of the cancer to be treated and also on the patient to be treated (age, weight, previous treatment(s), etc.).

Examples of cycles and doses are given in the study below.

An open-label, non-randomized, dose escalation, safety and pharmacokinetics phase I study of ombrabulin in combination with platinum salts (cisplatin or carboplatin) and taxanes (docetaxel or paclitaxel), every 3 weeks, in patients with advanced solid tumors has been done.

### STUDY OBJECTIVE(S)

### Primary objective

The primary objective of the study is to determine the recommended dose (RD) based on the incidence of dose limiting toxicity (DLT), the maximum administered dose (MAD), and the maximum tolerated dose (MTD) of ombrabulin in combination with platinum salts and taxanes, every 3 weeks in patients with advanced solid tumors for which platinum-taxane doublet has been approved or constitutes mainstay of care.

Thus, the primary endpoint of the study is:
- Dose limiting toxicity (DLT) at cycle 1.

### Secondary objectives

The secondary objectives of the study are:
- To assess the overall safety profile of the combination.
- To characterize at Cycle 1 the pharmacokinetic (PK) profile of ombrabulin given with platinum salts and taxanes following different schedules.
- To evaluate anti-tumor activity of the tritherapy combination.

Thus, the secondary endpoints of the study are:
- TEAE, post-TEAE, SAE and laboratory abnormalities.
- At cycle 1: pharmacokinetic (PK) parameters of ombrabulin given with platinum salts and taxanes following different schedules.
- Objective tumor response as defined by the RECIST criteria.

### STUDY DESIGN

Two groups of patients will be treated: one with docetaxel-cisplatin doublet (group 1) and the second with paclitaxel-carboplatin doublet (group 2), both in combination with ombrabulin.

The combination will be started with the following schedule (schedule A) for group 1:
Day 1: ombrabulin as a 30 minutes i.v. infusion immediately followed by 120 minutes i.v. infusion of cisplatin, and
Day 2: docetaxel administered as a 60 minutes i.v. infusion 24 hours apart ombrabulin infusion end
and for the first 4 dose levels (I, II, III, IV).

Cohorts of 3 or 6 patients will receive escalating doses of ombrabulin (15.5, 20 and 25 mg/m²) with a fixed dose of cisplatin at 75 mg/m² on Day 1, followed by docetaxel on Day 2, given either at 60 mg/m² for the ombrabulin doses of 15.5 and 20 mg/m² or at 75 mg/m² for the ombrabulin doses of 20 and 25 mg/m².

Taking into consideration the recommended dose of the combination ombrabulin and cisplatin administered the same day (25 mg/m² and 75 mg/m² respectively) every 3 weeks, after dose level IV, even if MAD not reached at this dose level, dose escalation of ombrabulin will be stopped and the combination will be administered with the following schedule (schedule B) in the 2 groups:
Day 1: ombrabulin as a 30 minutes i.v. infusion, and
Day 2:
Group 1: docetaxel administered as a 60 minutes i.v. infusion followed by cisplatin as a 120 minutes i.v. infusion, 24 hours apart ombrabulin infusion end.
Group 2: paclitaxel administered as a 180 minutes i.v. infusion followed by carboplatin as a 30 minutes i.v. infusion, 24 hours apart ombrabulin infusion end.

At each level, cohorts of 3 or 6 patients will receive escalating doses of ombrabulin (20, 25, 30, 35... mg/m²) followed at Day 2 by a fixed dose of cisplatin at 75 mg/m² or carboplatin AUC 5 or 6 in combination with docetaxel given at 75 mg/m² or paclitaxel either at 175 (regimen A) or 200 mg/m² (regimen B).

**Group 1 (ombrabulin/docetaxel/cisplatin):**

| **Dose escalation: ombrabulin with cisplatin (CDDP) and docetaxel (TXT)** | | | | |
|---|---|---|---|---|
| Schedule A | Dose- Levels | ombrabulin | CDDP | TXT |
| (ombrabulin and CDDP Day 1, TXT Day 2) | | mg/m² | mg/m² | mg/m² |
| | I | 15.5 | 75 | 60 |
| | II | 20 | 75 | 60 |
| | III | 20 | 75 | 75 |
| | IV | 25 | 75 | 75 |
| Schedule B | V* | 20 | 75 | 75 |
| (ombrabulin Day 1, CDDP and TXT Day 2) | VI | 25 | 75 | 75 |
| | VII | 30 | 75 | 75 |
| | VIII | 35 | 75 | 75 |

| | | | | |
|---|---|---|---|---|
| * if 2 DLTs at this dose level, possibility to test ombrabulin at 15.5 mg/m²- docetaxel 75 mg/m²- cisplatin 75 mg/m² | | | | |

**Group 2 (ombrabulin/paclitaxel/carboplatin):**

| **Dose escalation: ombrabulin with carboplatin (Cb) and paclitaxel (PXL): Regimen A** | | | | |
|---|---|---|---|---|
| Schedule B | Dose- Levels | ombrabulin | Cb | PXL |
| (ombrabulin Day 1, Cb and PXL Day 2) | | mg/m² (D1) | AUC (D2) | mg/m² (D2) |
| | Ia* | 20 | 5 | 175 |
| | *Ia'* | 20 | 6 | 175 |
| | IIa | 25 | 5 | 175 |
| | IIIa | 30 | 5 | 175 |
| | IVa | 35 | 5 | 175 |

| **Dose escalation: ombrabulin with carboplatin (Cb) and paclitaxel (PXL):** | | | | |
|---|---|---|---|---|
| **Regimen B** | | | | |
| Schedule B | Dose-Levels | ombrabulin | Cb | PXL |
| (ombrabulin Day 1, Cb and PXL Day 2) | | mg/m² (D1) | AUC (D2) | mg/m² (D2) |
| | Ib | 20 | 6 | 200 |
| | IIb | 25 | 6 | 200 |
| | IIIb | 30 | 6 | 200 |
| | IVb | 35 | 6 | 200 |

| | | | | |
|---|---|---|---|---|
| * if 2 DLTs at this dose level, possibility to test ombrabulin at 15.5 mg/m² NB: The first dose level to be tested in group 2 will be Ia, followed by Ia' then Ib. Then dose levels IIa-IIIa-IVa and IIb-IIIb-IVb could be run in parallel; dose escalation could be continued by increasing ombrabulin of 20% from previous dose, provided that tested dose levels had not shown 2 or more DLTs | | | | |

In group 2, dose escalation could be continued by increasing ombrabulin of 20% from previous dose for a maximum of 50 mg/m² (which is the recommended dose of the drug in monotherapy), provided that tested dose levels had not shown 2 or more DLTs.
In group 1, dose escalation will be stopped after dose level 35 mg/m² for ombrabulin, taking into account the recommended dose that has been reached with the bi-therapy (ombrabulin 35 mg/m² and docetaxel 75 mg/m²) in an on-going phase I trial.

Patients will then be followed for 21 days for safety assessment. After at least 21 days, patients will receive additional courses at every 21-day intervals in the absence of disease progression, unacceptable toxicity, or other study treatment criteria.
Thus, a cycle is defined as a 3 week-period including one ombrabulin, platinum salt and taxane administration.

Recruitment in groups 1 and 2 could be run in parallel. The first dose levels to be tested in group 2 will be:
- ombrabulin 20 mg/m²- Carboplatin AUC 5 - Paclitaxel 175 mg/m² (dose level la), followed by
- ombrabulin 20 mg/m²- Carboplatin AUC 6 - Paclitaxel 175 mg/m² (dose level la') followed by
- ombrabulin 20 mg/m²- Carboplatin AUC 6 - Paclitaxel 200 mg/m² (dose level Ib)
Then dose levels IIa-IIIa-IVa (regimen A) and IIb-IIIb-IVb (regimen B) could be run in parallel.

Once the MAD is reached in each group and regimen with schedule B, additional patients to complete a subset of at least 15 patients, will be treated at the immediate lower dose of ombrabulin with both platinum-taxane doublets chemotherapy (MTD) schedule B, mainly patients with non small cell lung cancer and ovarian cancer.

Cohorts of 3 or 6 patients will be screened and treated at each dose level. When the first three patients of a cohort have completed the first cycle, i.e. should have received at least one treatment course and should have been observed for acute toxicity for at least a 3-week follow-up period (or shorter period provided that a DLT has been observed), dose escalation strategy will be as follows:
In the absence of DLT at first cycle, three patients will be treated at the next dose level.
If DLT is observed at first cycle in 1 out of 3 patients, three further patients will be included at the same dose level and possibly at the same time. Then, if DLT is observed at first cycle in 1 out 6 patients, the next dose level will be tested. Otherwise, if 2 out 6 patients present with a DLT at first cycle, the MAD is considered to be achieved.
If DLT is observed at first cycle in 2 out of the 3 patients, the MAD is considered to have been reached.
The Maximum Administered Dose (MAD) will be reached at the dose at which ≥ 2 out of 3-6 patients develop a DLT at the first cycle.

The dose limiting toxicities ( DLTs) that are events to be watched and which allow to drive the escalation of dose, were predefined in the protocol in agreement with the scale of classification NCI-CTCAE version 3.

### Route(s) of administration:

ombrabulin, cisplatin, carboplatin, paclitaxel and docetaxel will be administered by intravenous infusion

### STUDY POPULATION

### Main inclusion criteria

- Advanced neoplastic disease (i.e. metastatic or locally advanced disease) for which platinum-taxane doublet regimens are approved or constitutes the mainstay of care such as non small cell lung cancer, epithelial ovary cancer, gastric cancer, transitional cell and bladder cancer and head and neck cancer.
- First or second line of metastatic disease.
- ≥ 18 years old.
- ECOG performance status of 0 to 1.

### Main exclusion criteria

- Related to the Methodology (concurrent treatment with any other anticancer therapy, absence of histologically or cytologically proven cancer at the first diagnosis, washout period of less than 3 weeks from prior anti-tumor therapy like chemotherapy, targeted agents, immunotherapy and radiotherapy or any investigational treatment, of less than 6 weeks from prior therapy with nitrosoureas or mitomycin).
- Related to the study drugs (previous carboplatin dose higher than 3000 mg/m² or cisplatin higher than 600 mg/m²; more than 1 line of previous chemotherapy as treatment for advanced cancer disease, neoadjuvant treatment excluded; severe hypersensitivity due to taxanes, polysorbate 80 and any other compound of the study combination; unadequate organ function including: neutrophils < 1.5 x 109/L; platelets < 100 x 109/L; creatinine ≥ 1.5 mg/dl, total bilirubin not within normal limit and ALT/AST/AP > 2.5 times the upper normal limits of the institutional norms).
- Cardiovascular exclusion criteria (documented medical history of myocardial infarction, documented angina pectoris, arrhythmia especially severe conduction disorder such as second or third-degree atrio-ventricular block, stroke, or history of arterial or venous thrombo-embolism within the past 6 months requiring anticoagulants; patient with a LVEF < 50% by echocardiography; patient with uncontrolled hypertension and patient with organ damage related to hypertension such as left ventricular hypertrophy or grade 2 ocular funduscopic changes or kidney impairment; 12-lead ECG: infarction Q-wave (at least in 2 contiguous derivations, duration > 40 msec, amplitude > 20% of QRS complex), ST segment depression or elevation ≥ 1 mm in at least 2 contiguous leads; untreated hypertension defined as systolic BP > 140 mmHg or diastolic BP > 90 mmHg on two repeated measurements at 30 minutes interval).

### RESULTS:

T (docetaxel D or paclitaxel P) and PS (cisplatin C or carboplatin Cb respectively)
Ob = ombrabulin
pts = patients
d = day

Forty-three patients (14 males and 29 females), median age 51 (range 24-74), including 25 chemonaive patients, were treated in 4 cohorts:
- Cohort I (Ob/C75 mg/m² d1, D60 or 75 mg/m² d2 - 13 pts)
- Cohort II (Ob d1, C75/D75 d2 -12 pts)
- Cohort III (Ob d1, CbAUC5/P175 d2 - 11 pts)
- Cohort IV (Ob d1, CbAUC6/P200 d2 - 7 pts).

Dose levels (DL) tested for Ob were: 15.5, 20, 25, 30, 35 mg/m².
Granulocyte growth factors were systematically administered as primary prophylaxis in cohort I and II.

The most common tumor types were lung (n=11), breast (n=11, including 3 triple negative pts) and ovarian (n=5).

Concerning cohort I:
- the median number of cycles was 6 (range 1-9);
- the RD is Ob20/C75/D75 mg/m²

Two DLTs (febrile neutropenia and pulmonary embolism) were reported at cycle 1 of dose levels 25/75/75 mg/m².
Other clinically significant grade (gr) 3/4 study drug related adverse events were: diarrhea, asthenia, drug hypersensitivity (2 pts each), transaminase increase, hypocalcemia, vomiting, nausea, peripheral neuropathy (1 pt each). Related cardiovascular events consisted on: gr 2 thrombo-phlebitis (3 pts), gr 2 left ventricular function decrease, gr 3 peripheral ischemia, gr 3 troponin increase and gr 2 hypertension (1 pt each). Hematotoxicity was typical for T and PS combinations.

Objective responses were observed: one complete response (patient with triple negative breast cancer), 7 partial responses (3 lung including one patient with squamous histology, 3 breast and 1 ovarian cancer) and 21 patients had stable disease.

Thus, these results confirm that the combination of Ob with T and PS is feasible and well tolerated, with preliminary encouraging evidence of anti-tumor activity.

## Claims

1. An antitumoral combination comprising ombrabulin, a taxane derivative and a platinum derivative, these therapeutic components being in the form of a free base or of an addition salt with a pharmaceutical acceptable acid, or in the form of a hydrate or of a solvate, where this antitumoral combination is well tolerated, does not exacerbate the toxicity of each of the antitumoral agents and which allows the treatment of advanced solid tumors either by stabilizing or by inducing a partial or a complete regression of the tumor.

2. Combination according to claim 1 where ombrabulin is in the form of the hydrochloride salt.

3. Combination according to claim 1 where the taxane derivative is chosen from paclitaxel or docetaxel.

4. Combination according to claim 1 where the platinum derivative is chosen from cisplatin or carboplatin.

5. Combination according to any one of claims 1 to 4 where ombrabulin is in combination with docetaxel and cisplatin or in combination with paclitaxel and carboplatin.

6. Combination according to any one of claims 1 to 5 comprising an effective quantity of ombrabulin, an effective quantity of a taxane derivative and an effective quantity of a platinum derivative.

7. Combination according to claim 6 where ombrabulin is administered at a dose comprised between 15 and 35 mg/m².

8. Combination according to claim 7 where ombrabulin is administered at a dose chosen from: 15.5; 20 ; 25 ; 30 and 35 mg/m².

9. Combination according to claim 6 where the taxane derivative is docetaxel and is administered at a dose of 60 or 75 mg/m².

10. Combination according to claim 6 where the taxane derivative is paclitaxel and is administered at a dose of 175 or 200 mg/m².

11. Combination according to claim 6 where the platinum derivative is cisplatin and is administered at a dose of 75 mg/m².

12. Combination according to claim 6 where the platinum derivative is carboplatin and is administered at a dose of AUC 5 or 6.

13. Combination according to claim 5 or 6 where ombrabulin is in combination with docetaxel and cisplatin and where ombrabulin is administered at a dose of 20 mg/m², docetaxel is administered at a dose of 75 mg/m² and cisplatin is administered at a dose of 75 mg/m².

14. Combination according to any one of claims 1 to 5, where the cycle of administration of the three antitumoral agents is repeated with an interval between two administrations of three weeks.

15. Use of ombrabulin, a taxane derivative and a platinum derivative for the preparation of an antitumoral combination as claimed in claims 1 to 14

16. An antitumoral pharmaceutical combination comprising ombrabulin, a taxane derivative and a platinum derivative, these agents being in the form of a free base or of an addition salt with a pharmaceutical acceptable acid, or in the form of a hydrate or of a solvate, where this antitumoral combination is well tolerated, does not exacerbate the toxicity of each of the antitumoral agents and which allows the treatment of advanced solid tumors either by stabilizing or by inducing a partial or a complete regression of the tumor, for its use as a medicament in the treatment of advanced solid tumors.
